Europäisches Patentamt

European Patent Office

Office européen des brevets

(19)

(11) Publication number: **0 069 913**

**B1**

(12) **EUROPEAN PATENT SPECIFICATION**

(45) Date of publication of patent specification: **25.03.87**

(51) Int. Cl.⁴: **A 61 B 1/12**

(21) Application number: **82105741.1**

(22) Date of filing: **29.06.82**

(54) Endoscope.

(30) Priority: **09.07.81 JP 107463/81**
**09.07.81 JP 107464/81**
**09.07.81 JP 107465/81**

(43) Date of publication of application:
**19.01.83 Bulletin 83/03**

(45) Publication of the grant of the patent:
**25.03.87 Bulletin 87/13**

(84) Designated Contracting States:
**DE**

(56) References cited:
**DE-A-2 920 724**
**DE-A-3 037 110**
**US-A-3 932 722**
**US-A-3 985 155**

(73) Proprietor: **OLYMPUS OPTICAL CO., LTD.**
**43-2, 2-chome, Hatagaya Shibuya-ku**
**Tokyo 151 (JP)**

(72) Inventor: **Yabe, Hisao**
**4-22-13, Oowada Hachioji-shi**
**Tokyo (JP)**

(74) Representative: **Kempe, Wolfgang, Dr.**
**Postfach 1273**
**D-6800 Mannheim 1 (DE)**

Courier Press, Leamington Spa, England.

## Description

The invention relates to an endoscope according to the preamble of claim 1, as disclosed in DE—A—2920724.

In general, an endoscope is provided with fluid passageways for introducing air, water or gas into a body cavity or for sucking a filthy liquid from the body cavity. The endoscope is also provided at the operating section with a switching device for selectively performing the air introducing operation, etc. The switching device consists of a cylinder and a piston. The piston, which is urged by a coil spring in a manner to project away from the cylinder, is held at its natural position, i.e., position at which stays the piston when the piston is not depressed, by a stopper. The air introducing operation, etc. are selectively performed by operating the piston.

It is necessary for the coil spring to be set as follows. Specifically, when the piston has been pushed into the cylinder, the coil spring should be capable of bringing the piston back to its natural position automatically against the resistance accompanying the sliding motion of various sealing members around the piston along the cylinder. It is also desirable that a comfortable repulsion be felt by the operator in depressing the piston. It should be noted in this connection that the piston is not depressed in introducing air into a body cavity; a leaking hole open at the head of the operating section is simply closed by a finger for the air introducing operation. Suppose the piston has been erroneously depressed, though it is intended to introduce air into the body cavity. In this case, the operator may not be aware of the error, if the repulsive force of the coil spring is too weak. However, if the repulsive force is too strong, the operator of the endoscope feels tired. It should be noted in this connection that the repulsive force of the coil spring is increased in proportion to the amount of the piston depression, with the result that the operator feels tired.

Washing and sterilization should also be considered. Since the coil spring is of complex spiral shape, it is necessary to use, for example, a brush for washing the coil spring mounted to the piston, leading to requirement of much labor. If the coil spring is detached from the piston, much labor is required for the detachment and mounting of the washed coil spring to the piston.

An object of this invention is to provide an endoscope comprising an urging member whose repulsive force does not increase in proportion to the amount of the piston depression. The urging member can be readily detached when the piston and cylinder are washed and sterilized.

To attain the above mentioned object, this invention provides an endoscope according to the features disclosed in the characterizing part of claim 1.

Further inventive features are disclosed in the sub-claims.

This invention can be more fully understood from the following detailed description when taken in conjunction with the accompanying drawings, in which:

Fig. 1 schematically shows an endoscope according to one embodiment of the present invention;

Fig. 2 is a cross sectional view showing the switching device included in the endoscope of Fig. 1;

Fig. 3 is a cross sectional view showing that the switching device of Fig. 2 is under the liquid introducing condition;

Fig. 4 is a graph showing the spring properties of a coil spring and an urging member used in the present invention;

Fig. 5 is a cross sectional view showing the switching device according to another embodiment of the present invention;

Fig. 6 is a cross sectional view showing the region around the urging member according to another embodiment of the present invention; and

Figs. 7 and 8 are cross sectional views each showing the region around the sealing member according to still another embodiment of the present invention.

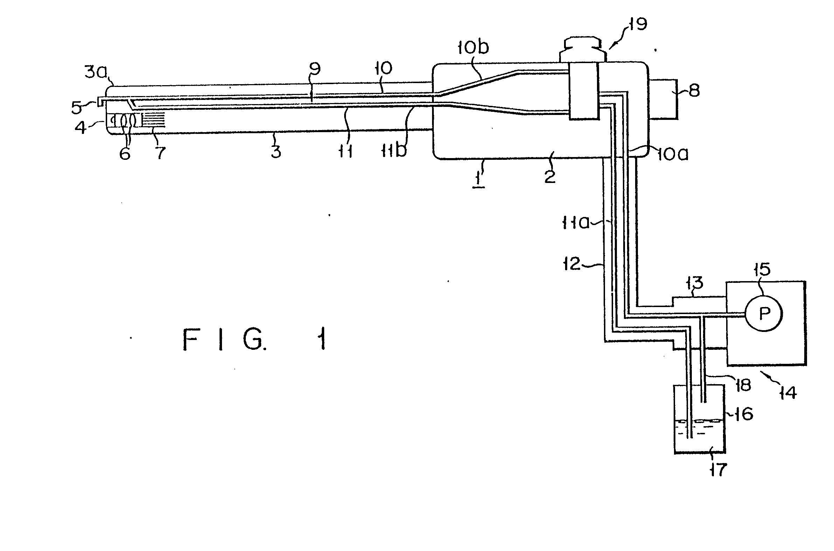

Fig. 1 schematically shows an endoscope 1 according to one embodiment of the present invention. As seen from the drawing, the endoscope 1 comprises an operating section 2 and an insert section 3 to be inserted into a body cavity.

The insert section 3 is provided at the distal end surface 3a with a view window 4 and a nozzle 5 with the open end facing the view window 4. The tip of an image guide 7 is positioned to face the view window 4 with a plurality of object lenses 6 provided between the tip and the view window 4. The image of the view field is formed by the object lenses 6 on the tip of the image guide 7. The image thus formed can be observed through the image guide 7 from a eyepiece section 8 included in the operating section 2.

Formed within the endoscope 1 is a fluid supply means 9 consisting of an air supply tube 10 and a liquid supply tube 11 each provided in both the operating section 2 and the insert section 3. These tubes 10 and 11 are joined each other near the distal end of the insert section 3, and the joined portion communicates with the nozzle 5. The proximal end portions of the tubes 10, 11 extend into a universal cord 12 having one end connected to the operating section 2. The other end of the universal cord 12 is connected via a connector 13 to an air-liquid supply unit 14 such that the air supply tube 10 is connected to an air pump 15 and the liquid supply tube 11 to a liquid supply tank 16. Further, a pressurizing tube 18 branched from the air supply tube 10 extends into the upper free space of the liquid supply tank 16 housing a washing liquid 17.

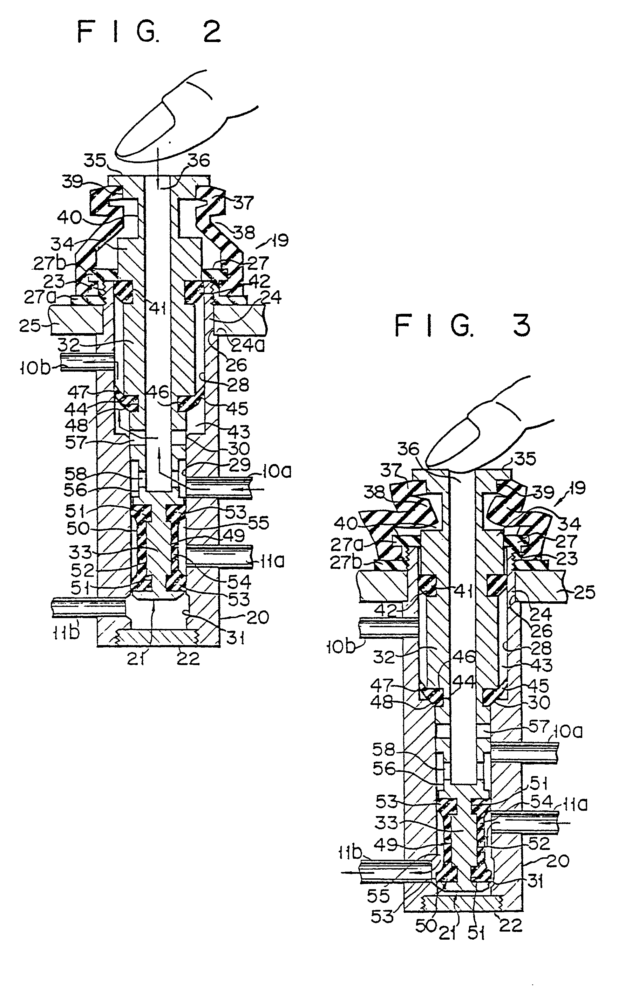

It is important to note that a switching device 19 for selectively introducing the air and liquid into a body cavity is mounted to the operating section 2. The switching device 19 comprises a cylinder 20 and a piston 21 as shown in Figs. 2 and 3. The lower open end of the cylinder 20 is closed by a bottom lid 22. The upper portion of the cylinder 20 is smaller in outer diameter than the other portion so as to form a step 24a. The outer surface in the upper portion of the cylinder 20 constitutes a mounting portion 24. A threaded portion 23 is included in the mounting portion 24. It should be noted that the mounting portion 24 is inserted into a hole 26 made in the wall 25 of the body of the operating section 2 such that the threaded portion 23 projects outward from the hole 26. Further, an internally threaded annular mounting member 27 is engaged with the threaded portion 23. The mounting member 27 comprises a lower flange 27a contacting the outer surface of the wall 25 and an upper flange 27b in direct contact with the upper surface of the cylinder 20. The upper flange 27b slightly extends toward the axis of the cylinder 20 so as to constitute a stopper. As seen from the drawing, the wall 25 is held between the lower flange 27a and the step 24a so as to mount the cylinder 20 to the wall 25. The inner diameter of the cylinder 20 is greater in the upper portion 28 than in the lower portion 29 and a tapered portion 30 is formed in the step between the upper and lower portions. Also, a circumferential groove 31 is formed in the lower portion 29 near the bottom lid 22. It should be noted that the cylinder 20 is disposed midway of each of the air supply tube 10 and the liquid supply tube 11 so as to divide these tubes into the upstream and downstream portions. The upstream portion 10a of the air supply tube 10 communicates with the inner space of the cylinder 20 at the upper part of the lower portion (small inner diameter portion) 29 of the cylinder 20, with the downstream portion 10b communicating with the inner space of the cylinder 20 about the middle of the upper portion 28 of the cylinder 20. On the other hand, the upstream portion 11a of the liquid supply tube 11 communicates with the inner space of the cylinder 20 about the middle of the lower portion 29 of the cylinder 20, with the downstream portion 11b communicating with the inner space of the cylinder 20 at the circumferential groove 31. As seen from the drawing, the upstream portion 11a is positioned lower than the upstream portion 10a of the air supply tube 10.

The piston 21 is inserted into the cylinder 20 of the construction described above. The piston 21 consists of a central portion 32, a lower portion 33 smaller in outer diameter than the central portion 32, and an upper portion 34 larger in outer diameter than the central portion 32. Under the ordinary condition, the upper portion 34 of the piston 21 is positioned outside the cylinder 20. The upper flat surface of the upper portion 34 acts as a finger-placing portion 35. It is seen that a leak hole 36 communicating with the outer space is formed within the piston 21 in a manner to extend upward from upper part of the lower portion 33. The piston 21 is urged upward by an urging member 37 of cover shape formed of an elastic material such as rubber. In the embodiment of Figs. 2 and 3, the urging member 37 is in the form of a cylinder having a relatively thick wall and comprises a central narrow portion 38 which is flared downwardly (a cylinder may be regarded as consisting of a plurality of covers joined together). If the piston 21 is depressed, the urging member 37 is folded at the central narrow portion 38. The lower end portion of the urging member 37 is elastically engaged detachably with the upper flange 27b of the mounting member 27. On the other hand, the upper end portion of the urging member 37 is elastically engaged detachably with a recess 39 made in the lower portion of the finger-placing portion 35. Thus, if the piston 21 is depressed by a finger as shown in Fig. 3, the urging member 37 is elastically folded at the central narrow portion 38 and the piston 21 is inserted into the cylinder 20 against the restoring force of the urging member 37. A circumferential groove 40 is formed in the outer surface of the upper portion 34 of the piston 21. If the piston 21 is depressed, the folded portion of the urging member 37 is housed in the groove 40. Thus, the folded portion is not pushed against the piston 21. Nor is the folded portion projected outward.

A first mounting groove 41 is formed along the outer circumference of the piston 21 at the boundary between the upper portion 34 and the central portion 32. A first annular sealing member 42 formed of an elastic material such as silicone rubber is mounted to the first mounting groove 41. Under the ordinary condition as shown in Fig. 2, i.e., when the piston is not depressed, the first sealing member 42 abuts against the bottom surface of the upper flange 27b of the mounting member 27 so as to prevent the piston 21 from being projected from the cylinder 20 by the bias force of the urging member 37. The first sealing member 42 hermetically abuts against the upper flange 27b so as to keep hermetic an air passageway 43 formed between the inner surface of the cylinder 20 and the outer surface of the piston 21 in the upper portion of the piston-cylinder assembly. It should be noted that, if the piston 21 is pulled upward, the first sealing member 42 is elastically deformed so as to permit the piston 21 to be withdrawn from the cylinder 20.

A second mounting groove 44 is formed along the outer circumference of the piston 21 at the boundary between the central portion 32 and the lower portion 33. An annular valve means 45 formed of an elastic material is mounted to the second mounting groove 44. The valve means 45 consists of a base portion 46 housed within the second mounting groove 44 and a flexible portion 47 extending obliquely upward from the base portion 46 such that the tip of the flexible portion 47 is in direct contact with the inner surface of the cylinder 20. The base portion 46 includes an inclined surface 48. If the piston 21 is depressed as shown in Fig. 3, the inclined surface 48 is brought into tight contact with the tapered portion 30 formed in the step between the upper portion 28 and the lower portion 29 of the cylinder 20. The flexible portion 47 serves to define the flowing direction of the air within the air passageway 43. Specifically, when the air flows upward within the passageway 43, the flexible portion 47 is elastically bent upward by the air pressure so as to provide a clearance between the tip of the flexible portion 47 and the inner surface of the cylinder 20, thereby permitting the upward air flow within the air passageway 43. But, the valve means 45 is closed if the air pressure is applied downwardly to the flexible portion 47 as apparent from the drawing.

A third mounting groove 49 is formed along the outer circumference of the lower portion 33 of the piston 21. A second sealing member 50, which is substantially cylindrical, is mounted to the third mounting groove 49. The third mounting groove 49 consists of a pair of side grooves 51 apart from each other in the axial direction of the piston 21 and a shallow central groove 52 extending between the two side grooves 51. On the other hand, the second sealing member 50 consists of a pair of projections 53 corresponding to the side grooves 51 of the third mounting groove 49 and a thin central portion 54 corresponding to the central groove 52 of the third mounting groove 49. As seen from the drawing, the outer surface of the projection 53 is in direct contact with the inner surface of the lower portion 29 of the cylinder 20. But, a clearance 55 is provided between the outer surface of the central portion 54 of the second sealing member 50 and the inner surface of the lower portion 29 of the cylinder 20. When the piston 21 is in the natural position, the communication between the upstream portion 11a and the downstream portion 11b of the liquid supply tube 11 is broken by the projection 53 of the second sealing member 50 as seen from Fig. 2. If the piston 21 is depressed, the upstream portion 11a is allowed to communicate with the downstream portion 11a via the clearance 55 as seen from Fig. 3.

A communication groove 56 is also formed along the outer circumference of the lower portion 33 of the piston 21. As seen from the drawing, the groove 56 is positioned above the third mounting groove 49 and is long enough in the vertical direction to permit the upstream portion 10a of the air supply tube 10 to communicate with the groove 56 whether the piston 21 is in the natural position as shown in Fig. 2 or in depressed as shown in Fig. 3. Further, the piston 21 is provided at the lower portion 33 with a first through-hole 57 and a second through-hole 58. When the piston 21 is in the natural position, the leak hole 36 is allowed to communicate with the air passageway 43 via the first through-hole 57 as seen from Fig. 2. On the other hand, the second through-hole 58 serves to allow the communication groove 56 to communicate with the leak hole 36.

The endoscope of the construction described above is operated as described in the following. If the air pump 15 is operated with the leak hole 36 left open to the outside, the air forwarded to the upstream portion 10a of the air supply tube 10 enters the leak hole 36 through the communication groove 56 and the second through-hole 58. Then, the air is released to the atmosphere through the leak hole 36 because the flow resistance in the leak hole 36 is markedly lower than that in the downstream portion 10b leading to the nozzle 5 at the distal end of the insert section 3. If the open end of the leak hole 36 is closed by a finger, however, the air entering the leak hole 36 flows into the downstream portion 10b of the air supply tube 10 through the first through-hole 57 and the air passageway 43 as denoted by arrows in Fig. 2. In this case, the flexible portion 47 of the valve means 45 is upwardly bent by the air pressure to permit the air flow into the downstream portion 10b as mentioned previously. It follows that the air supplied for the air pump 15 is released through the nozzle 5 mounted at the distal end of the insert section 3.

For performing a liquid supply operation, the piston 21 is depressed, with the leak hole 36 closed by a finger, such that the inclined surface 48 of the valve means 45 abuts against the tapered portion 30 of the cylinder 20 as shown in Fig. 3. If the piston 21 is depressed in this fashion, the communication between the upstream portion 10a and the downstream portion 10b of the air supply tube 10 is broken by the hermetic contact of the inclined plane 48 with the tapered portion 30, with the result that the air discharged from the air pump 15 flows into the pressurizing tube 18 so as to pressurize the interior of the liquid tank 16. It follows that the washing liquid within the liquid tank 16 is allowed to flow into the upstream portion 11a of the liquid supply tube 11. It should be noted that the upstream portion 11a is allowed to communicate with the downstream portion 11b via the clearance 55 if the piston 21 is depressed as shown in Fig. 31 (see arrows). To be brief, if the piston 21 is depressed with the open end of the leak hole 36 closed as shown in Fig. 3, the washing liquid supplied from the liquid tank 16 is spurted into a body cavity through the nozzle 5.

It is important to pay attentions to the function of the urging member 37 accompanying the depression of the piston 21. Specifically, the entire region of the urging member 37 is elastically deformed in the initial stage of the piston depression, allowing the operator to feel a comfortable repulsive force. But, if the amount of depression exceeds a certain level, the narrow central portion 38 of the urging member 37 is folded inward, with the result that the repulsive force of the urging member 37 scarcely increases in spite of the progress of the piston insertion into the cylinder 20. Thus, the piston 21 can be depressed with small force.

Also, the urging member 37 is of cover shape, rendering it possible to wash easily the outer surface of the urging member 37. Further, the urging member 37, if simply deformed elastically, is readily disengaged from the mounting member 27 and the finger-placing portion 35 so as to be detached from the piston 21. Naturally, both the inner and outer surfaces of the detached urging member 37 can be washed easily.

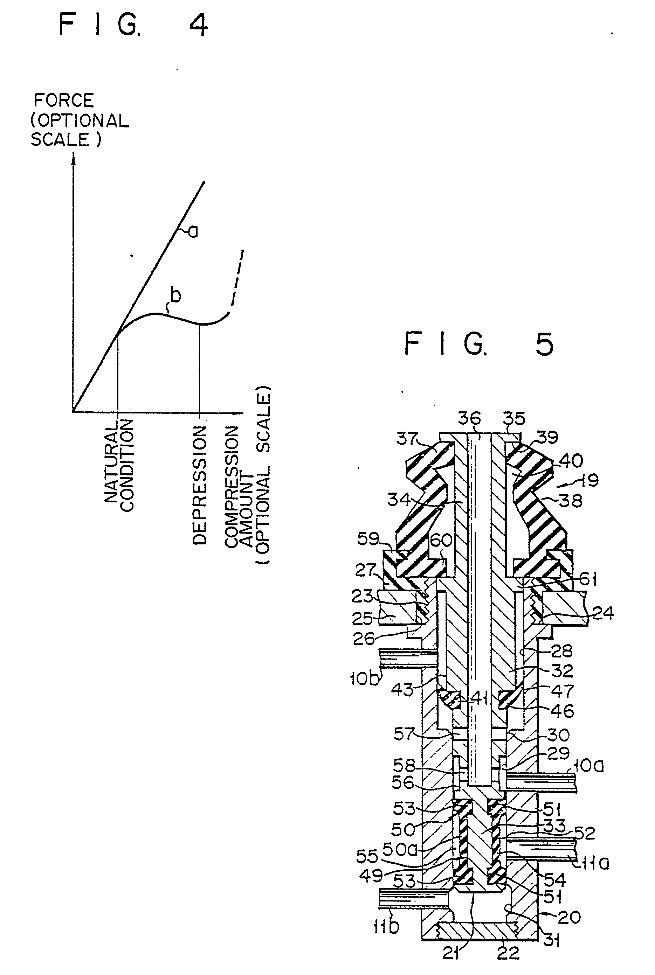

Fig. 4 shows the changes in the compression amounts of a coil spring and the urging member 37 of the present invention relative to the force applied thereto, curves a and b shown therein representing the coil spring and the urging member 37, respectively. It is seen that the compression amount is substantially proportional to the force applied to the coil spring (see curve a). In the case of the urging member 37 (curve b), however, it is certainly necessary to increase the force in accordance with increase in the compression amount before the central narrow portion 38 begins to be folded. But, the force scarcely increases after the central narrow portion 38 begins to be folded. As seen from Fig. 4, the coil spring and the urging member 37 are set equal to each other in the repulsive force under the natural position of the piston, i.e., when the piston 21 is not depressed.

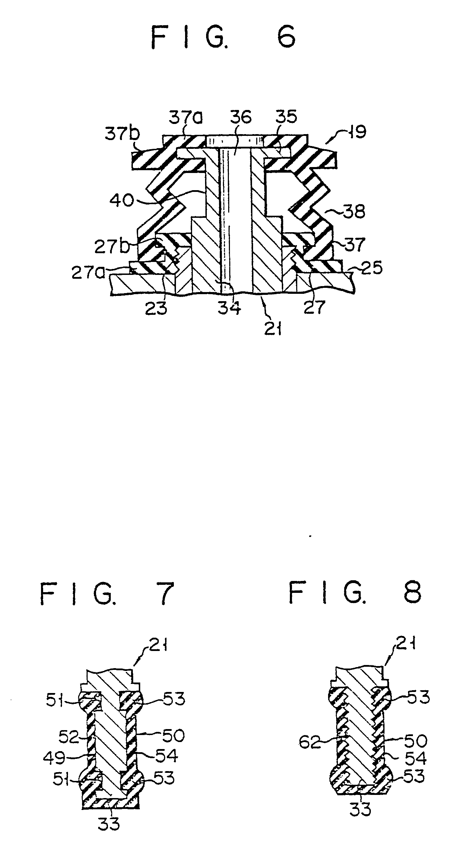

Fig. 5 shows a modification of the switching device shown in Figs. 2 and 3. In the modification of Fig. 5, the mounting member 27 is provided with a collar 59 extending upward from the upper surface of the wall 25 of the body of the operating section 2 and, thus, bent inward at substantially right angles so as to define a recess. The lower end portion of the urging member 37 is engaged with the recess of the collar 59 and is provided along the inner circumference with a flange 60 extending toward the axis of the cylinder 20 and acting as a stopper. Also, the housing groove 40 extends over the entire length of the upper portion 34 of the piston 21 except the finger-placing portion 35. Further, an annular projection 61 is provided in place of the first sealing member 42 at the boundary between the upper portion 34 and the central portion 32 of the piston 21. In this construction, the central narrow portion 38 of the urging member 37 is folded inward if the amount of piston depression exceeds a certain level, with the result that the liquid supply operation can be performed with small force.

The urging member 37 may also be formed as shown in Fig. 6. Specifically, the upper portion of the urging member 37 is provided with an engaging portion 37a having an annular recess and with an annular knob 37b extending outward from the engaging portion 37a. As seen from the drawing, the finger-placing portion 35 is engaged with the recess of the engaging portion 37a so as to ensure the engagement therebetween. The knob 37b facilitates the disengagement of the engaging portion 37a from the finger-placing portion 35. The construction of Fig. 6 also permits producing the particular effect of the urging member 37 described previously. In addition, a finger is prevented from slipping in depressing the piston 21 because the finger-placing portion 35 is covered with the engaging portion 37a. Also, the engaging portion 37a is felt pleasant.

In any of the embodiments described above, the urging member 37 is elastically deformed upon depression of the piston 21 so as to allow the central narrow portion 38 to be folded inward. Since the folded portion is housed in the housing groove 40 formed in the upper portion 34 of the piston 21, the urging member 37 is scarcely projected outward. Thus, where the operating section 2 is provided with a plurality of switching devices 19 for the gas supply operation, sucking operation, etc. as well as for the air-liquid supply operation described above, it is possible to dispose the switching devices 19 very close to each other, because it is impossible for the adjacent urging members 37 to be brought into mutual contact and, thus, the piston 21 can be depressed with small force.

In order to withdraw the piston 21 from the cylinder 20 for washing and sterilizing the piston 21 and the interior of the cylinder 20, the lower end of the urging member 37 is disengaged from the mounting member 27 and, then, the piston 21 is strongly pulled upward. In the embodiment of Figs. 2 and 3, the first sealing member 42 is elastically deformed by the pulling of the piston 21 so as to be disengaged from the stopper, i.e., the upper flange 27b of the mounting member 27. The flexible portion 47 of the valve means 45 is also deformed elastically when brought into contact with the upper flange 27b. Thus, the piston 21 can be readily withdrawn from the cylinder 20. After the washing and sterilization of the cylinder 20 and the piston 21, the piston 21 is inserted into the cylinder 20 by strongly pushing the piston 21. As in the withdrawing step, the flexible portion 47 of the valve means 45 and the first sealing member 42 are elastically deformed when brought into contact with the upper flange 27b of the mounting member 27, if the piston 21 is strongly pushed, so as to permit the piston 21 to be inserted into the cylinder 20. To be brief, the piston 21 can be withdrawn from and inserted into the cylinder 20 with small force, because it suffices to deform elastically the first sealing member 42 for performing the withdrawing-inserting operation of the piston 21. In the embodiment of Fig. 5, the flange 60 of the urging member 37 is elastically deformed by the annular projection 61 when the piston 21 is withdrawn from or inserted into the cylinder 20 so as to permit the withdrawing-inserting of the piston 21. Thus, the withdrawing-inserting of the piston 21 can be performed with small force, because it suffices to deform elastically the flange 60 of the urging member 37 for performing the withdrawing-inserting operation.

In the embodiments described above, the stopper, e.g., upper flange 27b of the mounting member 27, is made of a rigid material, with the abutting member, e.g., first sealing member 42, made of an elastic material. But, the particular effect described above can be obtained if at least one of the stopper and the abutting member is made of an elastic material.

Under the ordinary condition, the piston 21 is prevented from projecting away from the cylinder 20 by the abutment of the abutting member against the stopper. But, the piston 21 can be withdrawn from the cylinder 20 by simply pulling the piston 21 with force simply large enough to deform elastically the elastic stopper or abutting member. Likewise, the piston 21 can be inserted into the cylinder 20 with force simply large enough to deform elastically the elastic member mentioned. To be brief, the piston 21 can be detached from or mounted to the cylinder 20 easily, rendering it possible to wash and sterilize the piston 21 and the cylinder 20 easily and promptly.

It should be noted that the communication groove 56 formed in the piston 21 is allowed to communicate with the upstream portion 10a of the air supply tube 10 under both the air supply condition as shown in Fig. 2 and the liquid supply condition as shown in Fig. 4. This is advantageous in that the air is not abnormally introduced into a body cavity. Suppose the urging member 37 has failed to be mounted inadvertently to the switching device 19 after the sterilization. In this case, the piston 21 is brought into the depressed position as shown in Fig. 3. However, the air discharged from the air pump 15 is released to the atmosphere from the leak hole 36 via the upstream portion 10a of the air supply tube 10, the communication groove 56 and the second through-hole 58, because the flow resistance of the above-noted system is markedly lower than that of pressurizing tube 18 leading to the liquid supply tank 16. Thus, the air is not abnormally introduced into a body cavity as mentioned above.

The second sealing member 50 is formed as an integral cylindrical body consisting of the paired projections 53 and the thin central portion 54, and is mounted to the third mounting groove 49. Thus, the second sealing member 50 is substantially immovable within the third mounting groove 49 regardless of the movement of the piston 21 relative to the cylinder 20. Naturally, the third mounting groove 49 is not stained at all, rendering it unnecessary to remove the second sealing member 50 from the third mounting groove 49 in washing and sterilizing the piston 21. It should also be noted that the projections 53 of the second sealing member 50 are engaged with the side grooves 51 of the third mounting groove 49. Thus, even if the vertical dimension of the third mounting groove 49 is somewhat inaccurate, the inaccuracy is absorbed by the elastic deformation of the thin central portion 54 of the second sealing member 50, with the result that the second sealing member 50 can be mounted to the third mounting groove 49 without fail. In other words, the second sealing member 50 is enabled to perform its sealing function satisfactorily if the side grooves 51 of the third mounting groove 49 are formed accurate in depth. It follows that the second sealing member 50 need not be removed from the third mounting groove 49 in washing the piston 21. Naturally, the piston 21 can be washed efficiently. Further, since the elastic deformation of the central narrow portion 54 permits thesecond sealing member 50 to have a degree of freedom in the vertical dimension thereof, it suffices to control accurately the thickness alone of the second sealing member 50. It follows that the third mounting groove 49 can be formed easily.

As described previously, the circumferential groove 31 is formed near the bottom of the cylinder 20. Also, the downstream portion 11b of the liquid supply tube 11 communicates with the inner space of the cylinder 20 at the groove 31. It should be noted that, if the piston 21 is depressed for the liquid supply operation, the lower projection 53 of the second sealing member 50 is positioned to face the groove 31. Naturally, a clearance is provided between the lower projection 53 and the groove 31. Thus, even if the downstream portion 11b of the liquid supply tube 11 somewhat projects into the cylinder 20, the lower projection 53 is not damaged by the projecting edge of the downstream portion 11b.

Fig. 7 shows a modification of the second sealing member 50. In this modification, the second sealing member 50 extends to cover the lower end of the piston 21, with the result that a filthy material is prevented from entering the third mounting groove 49 without fail. Fig. 8 shows another modification. In this case, a threaded portion 62 is formed in place of the third mounting groove 49 in the lower end portion of the piston 21. What should be noted is that the second sealing member 50 is formed to cover the threaded portion 62 and the lower end of the piston 21 by insert molding, with the threaded portion 62 used as the insert.

Needless to say, the switching device of the construction described above can be used for the gas supply-suction switching operation as well as for the air supply-liquid supply switching operation.

## Claims

1. Endoscope with an insert section (3) including a distal end portion insertable into a coeliac cavity and a proximal end portion connected with an operating section (2), fluid supply passages (9) provided in the insert section (3) and the operating section (2), as well as a switching device (19) mounted to the operating section (2) for controlling the fluid flow through the fluid supply passages (9), the switching device (19) comprising a cylinder (20) communicating with an intermediate portion of the fluid supply passages (9) and being provided at least with one open end in the axial direction into which a piston (21) is inserted, the outer end portion of the piston (21) being positioned outside of the cylinder (20) while the portion of the piston (21) inserted in the cylinder (20) selectively opens and closes the fluid supply passages (9), and an elastic urging member (37) for urging the piston outwards of the cylinder, characterized in that the urging member (37) is of cover shape for covering the open end of the cylinder and is mounted between the open end of the cylinder (20) and the outer end portion of the piston (21), said urging member being connected with a mounting flange (27b) provided at the open end of the cylinder (20), in that the urging member (37) is substantially in the shape of the cylinder (20), is opened at both ends, comprising a narrow central portion (38), and has the outer end portion thereof engaged with the outer circumference of the outer end portion of the piston (21), and in that the central portion of the urging member (37) folds elastically when the piston (21) is depressed and is bent with respect to its longitudinal axis when the piston (21) is in the natural position.

2. The endoscope according to claim 1, wherein the upper portion of the piston (21) is provided along the outer circumference thereof with a groove (40) for housing the narrow central portion (38) of the urging member (37) when the narrow central portion (38) is folded inward.

3. The endoscope according to claim 1, wherein the cylinder (20) is provided at the open end portion thereof with an inwardly extending stopper (27, 60) and the piston (21) is provided at the inserted portion thereof with a member (42, 61) abutting against the stopper (27, 60) when the piston (21) is in the natural position, at least one of the stopper (27, 60) and the abutting member (42, 61) being formed of an elastic material.

4. The endoscope according to claim 3, wherein the abutting member (42, 61) is formed along the outer circumference of the piston (21) and slides along the inner surface of the cylinder (20) so as to perform a sealing function.

5. The endoscope according to claim 3, wherein a mounting member (27) for fixing the cylinder (20) to the body of the operating section (2) is provided with the stopper (27, 60).

6. The endoscope according to claim 3, wherein the urging member is provided with the stopper (27, 60).

7. The endoscope according to claim 1, wherein the piston (21) is provided with a leak hole (36) open to the outside, the fluid supply means (9) comprises an air passageway (10), the upstream portion of said air passageway (10) extending through the wall of the cylinder (20), and the piston (21) is provided with a communication groove permitting the upstream portion (10a) of the air passageway (10) to communicate with the leak hole (36) whether the piston (21) is in the natural position or is depressed.

8. The endoscope according to claim 1, wherein the lower end portion of the piston (21) is covered with a cylindrical sealing member (50) consisting of a pair of projections (53) and a thin central portion (54), the outer surfaces of the projections (53) directly contacting the inner surface of the cylinder (20) so as to provide a clearance (55) defined by the projections (53), the outer surface of the thin central portion (54) of the sealing member and the inner surface of the cylinder (20), said clearance (55) allowing the upstream portion (11a) of a fluid passageway (11) to communicate with the downstream portion (11b) of the fluid passageway (11) when the piston (21) is in the depressed position, and one of the projections (53) breaking the liquid passageway (11) when the piston (21) is in the natural position.

9. The endoscope according to claim 8, wherein the cylindrical sealing member (50) is open at both ends and is in tight contact with the outer surface of the piston (21).

10. The endoscope according to claim 8, wherein the sealing member (50) extends to cover the lower end surface of the piston (21).

11. The endoscope according to claim 8, wherein the lower portion of the piston (21) is provided along the outer circumference thereof with a groove (49) receiving the sealing member (50).

12. The endoscope according to claim 8, wherein the sealing member (50) is formed by insert molding, with the lower end portion of the piston (21) used as the insert.

**Patentansprüche**

1. Endoskop mit einem Einführabschnitt (3), der ein distales Endstück zum Einführen in eine Körperhöhle und ein proximales Endstück, das mit einem Bedienungsabschnitt (2) verbunden ist, aufweist, mit Flüssigkeitszuleitungen (9) im Einführabschnitt (3) und im Bedienungsabschnitt (2) sowie mit einer Schaltvorrichtung (19) am Bedienungsabschnitt (2) zur Steuerung des Flüssigkeitsflusses durch die Flüssigkeitszuleitungen (9), die einen Zylinder (20), der mit einem mittleren Abschnitt der Flüssigkeitszuleitungen (9) in Verbindung steht und mit wenigstens einem offenen Ende in axialer Richtung versehen ist, in das ein Kolben (21) eingesetzt ist, dessen äußerer Endabschnitt aus dem Zylinder (20) herausragt, während der in den Zylinder (20) eingeführte Abschnitt des Kolbens (21) wahlweise die Flüssigkeitszuleitungen (9) öffnet oder schließt, und ein elastisches Zwangsglied (37) aufweist, das den Kolben aus dem Zylinder herausdrückt, dadurch gekennzeichnet, daß das Zwangsglied (37) die Form einer Kappe besitzt, die das offene Ende des Zylinders bedeckt und zwischen dem offenen Ende des Zylinders (20) und dem äußeren Endabschnitt des Kolbens (21) angebracht ist, wobei das Zwangsglied mit einem Befestigungsflansch (27b) am offenen Ende des Zylinders (20) verbunden ist, daß das Zwangsglied (37) im wesentlichen der Form des Zylinders (20) folgt, an beiden Enden offen ist, einen engen Zentralabschnitt (38) aufweist, dessen nach außen weisendes Ende mit dem Außenumfang des äußeren Endabschnittes des Kolbens (21) in Eingriff steht, und daß sich der Zentralabschnitt des Zwangsgliedes (37) elastisch zusammenfaltet, wenn der Kolben (21) niedergedrückt wird, und in bezug auf seine Längsachse durchgebogen ist, wenn sich der Kolben (21) in seiner Ausgangsstellung befindet.

2. Endoskop nach Anspruch 1, dadurch gekennzeichnet, daß der äußere Endabschnitt des Kolbens (21) entlang seines Außenumfanges eine Nut (40) aufweist, die den engen Zentralabschnitt (38) des Zwangsgliedes (37) aufnimmt, wenn der enge Zentralabschnitt (38) nach innen gefaltet ist.

3. Endoskop nach Anspruch 1, dadurch gekennzeichnet, daß der Zylinder (20) an seinem offenen Endabschnitt, einen sich nach innen erstreckenden Anschlag (27, 60) und der Kolben (21) an seinem eingeführten Abschnitt ein Bauteil (42, 61) aufweist, das an den Anschlag (27, 60) anstößt, wenn sich der Kolben in seiner Ausgangsstellung befindet, wobei mindestens der Anschlag (27, 60) oder das Bauteil (42, 61) aus einem elastischen Werkstoff besteht.

4. Endoskop nach Anspruch 3, dadurch gekennzeichnet, daß das Bauteil (42, 61) entlang des Außenumfanges des Kolbens (21) angeordnet ist und an der Innenfläche des Zylinders (20) gleitet, so daß es eine Dichtungsfunktion ausübt.

5. Endoskop nach Anspruch 3, dadurch gekennzeichnet, daß der Anschlag (27, 60) ein Befestigungsteil (27) zur Befestigung des Zylinders (20) an dem Gehäuse des Bedienungsabschnittes (2) aufweist.

6. Endoskop nach Anspruch 3, dadurch gekennzeichnet, daß das Zwangsglied den Anschlag (27, 60) aufweist.

7. Endoskop nach Anspruch 1, dadurch gekennzeichnet, daß der Kolben (21) eine nach außen hin offene Bohrung (36) aufweist, daß die Flüssigkeitszuleitung (9) mit einer Luftdurchführung (10) versehen ist, deren oberstromiger Abschnitt sich durch die Wandung des Zylinders (20) erstreckt, und daß der Kolben eine Verbindungsnut aufweist, durch die der oberstromige Abschnitt (10a) der Luftdurchführung (10) mit der Bohrung (36) kommunizieren kann, wenn, sich der Kolben (21) in seiner Ausgangsstellung befindet oder niedergedrückt ist.

8. Endoskop nach Anspruch 1, dadurch gekennzeichnet, daß das untere Endstück des Kolbens (21) mit einer zylindrischen Dichtung (50) bedeckt ist, die aus einem Paar von Vorsprüngen (53) und einem dünnen Zentralteil (54) besteht, daß die Außenflächen der Vorsprünge (53) die Innenfläche des Zylinders (20) direkt kontaktieren, so daß ein durch die Vorsprünge (53), die Außenfläche des dünnen Zentralteiles (54) der Dichtung und die Innenfläche des Zylinders (20) ein definierter Zwischenraum (55) gebildet wird, der dem oberstromigen Abschnitt (11a) einer Flüssigkeitsdurchführung (11) ermöglicht, mit dem unterstromigen Abschnitt (11b) der Flüssigkeitsdurchführung (11) zu kommunizieren, wenn sich der Kolben (21) in der niedergedrückten Stellung befindet, und daß einer der Vorsprünge (53) die Flüssigkeitsdurchführung (11) unterbricht, wenn sich der Kolben (21) in seiner Ausgangsstellung befindet.

9. Endoskop nach Anspruch 8, dadurch gekennzeichnet, daß die zylindrische Dichtung (50) an beiden Enden offen ist und in eng anliegendem Kontakt mit der Außenfläche des Kolbens (21) ist.

10. Endoskop nach Anspruch 8, dadurch gekennzeichnet, daß die Dichtung (50) die untere Stirnfläche des Kolbens (21) bedeckt.

11. Endoskop nach Anspruch 8, dadurch gekennzeichnet, daß der untere Endabschnitt des Kolbens (21) an seiner äußeren Umfangsfläche eine Nut (49) zur Aufnahme der Dichtung (50) aufweist.

12. Endoskop nach Anspruch 8, dadurch gekennzeichnet, daß die Dichtung (50) durch Spritzguß hergestellt ist, wobei das untere Endstück des Kolbens (21) als Formteil benutzt ist.

**Revendications**

1. Endoscope muni d'une section d'insertion (3) comprenant une partie terminale distale pouvant être insérée dans une cavité coeliaque et une partie terminale proximale raccordée à une section de commande (2), des passages (9) d'alimentation en fluide formés dans la section d'insertion (3) et dans la section de commande (2), ainsi qu'un dispositif de commutation (19) monté sur la section de commande (2) pour commander l'écoulement du fluide à travers les passages (9) d'alimentation en fluide, le dispositif de commutation (19) comprenant un cylindre (20) communiquant avec une partie intermédiaire des passages (9) d'alimentation en fluide et pourvu dans la direction axiale d'au moins une extrémité ouverte dans laquelle est inséré un piston (21), la partie terminale extérieure du piston (21) étant placée à l'extérieur du cylindre (20), tandis que la partie du piston (21) insérée dans le cylindre (20) ouvre et ferme sélectivement les passages (9) d'alimentation en fluide, et un élément de poussée élastique (37) destiné à pousser le piston vers l'extérieur du cylindre, caractérisé en ce que l'élément de poussée (37) a la forme d'un capot pour recouvrir l'extrémité ouverte du cylindre et est monté entre l'extrémité ouverte du cylindre (20) et la partie terminale extérieure du piston (21), ledit élément de poussée étant assemblé à un rebord de montage (27b) formé à l'extrémité ouverte du cylindre (20), en ce que l'élément de poussée (37) a sensiblement la forme du cylindre (20), est ouvert à ses deux extrémités, comprend une partie centrale resserrée (38), et est en prise à son autre partie terminale avec la circonférence extérieure de la partie terminale extérieure du piston (21), et en ce que la partie centrale de l'élément de poussée (37) se plie élastiquement quand on enfonce le piston (21) et est coudée par rapport à son axe longitudinal quand le piston (21) se trouve dans la position naturelle.

2. Endoscope selon la revendication 1, dans lequel la partie supérieure du piston (21) comporte le long de sa circonférence extérieure une gorge (40) destinée à loger la partie centrale resserrée (38) de l'élément de poussée (37) lorsque la partie centrale resserrée (38) est pliée vers l'intérieur.

3. Endoscope selon la revendication 1, dans lequel le cylindre (20) comporte à sa partie terminale ouverte un élément d'arrêt (27b, 60) s'étendant vers l'intérieur et le piston (21) est pourvu sur sa partie insérée d'un élément de butée (42, 61) portant contre l'élément d'arrêt (27b, 60) quand le piston (21) se trouve dans la position naturelle, au moins l'un des éléments d'arrêt (27b, 60) et de butée (42, 61) étant formé d'une matière élastique.

4. Endoscope selon la revendication 3, dans lequel l'élément de butée (42, 61) est formé le long de la circonférence extérieure du piston (21) et coulisse le long de la surface inférieure du cylindre (20), de manière à remplir une fonction d'étanchéité.

5. Endoscope selon la revendication 3, dans lequel un élément de montage (27) destiné à fixer le cylindre (20) au corps de la section de commande (2) est pourvu d'un élément d'arrêt (27b, 60).

6. Endoscope selon la revendication 3, dans lequel l'élément de poussée est muni de l'élément d'arrêt (27b, 60).

7. Endoscope selon la revendication 1, dans lequel le piston (21) est pourvu d'un trou de fuite (36) ouvert vers l'extérieur, le moyen (9) d'alimentation en fluide comprenant un passage (10) d'air, la partie d'amont dudit passage (10) d'air s'étendant à travers la paroi du cylindre (20), et le piston (21) étant pourvu d'une gorge de communication permettant à la partie d'amont (10a) du passage (10) d'air de communiquer avec le trou de fuite (36), que le piston (21) soit dans la position naturelle ou qu'il soit enfoncé.

8. Endoscope selon la revendication 1, dans lequel la partie terminale inférieure du piston (21) est recouverte d'un élément cylindrique d'étanchéité (50) consistant en une paire de saillies (53) et en une mince partie centrale (54), les surfaces extérieures des saillies (53) étant directement en contact avec la surface inférieure du cylindre (20) de manière à assurer un espace libre (55) défini par les saillies (53), la surface extérieure de la mince partie centrale (54) de l'élément d'étanchéité et la surface intérieure du cylindre (20), ledit espace libre (55) permettant à la partie d'amont (11a) d'un passage (11) de fluide de communiquer avec la partie d'aval (11b) du passage (11) de fluide lorsque le piston (21) se trouve dans la position enfoncée, et une des saillies (53) obturant le passage (11) de liquide quand le piston (21) se trouve dans la position naturelle.

9. Endoscope selon la revendication 8, dans lequel l'élément cylindrique d'étanchéité (50) est ouvert à ses deux extrémités et se trouve en contact serré avec la surface extérieure du piston (21).

10. Endoscope selon la revendication 8, dans lequel l'élément d'étanchéité (50) s'étend de manière à recouvrir la surface d'extrémité inférieure du piston (21).

11. Endoscope selon la revendication 8, dans lequel la partie inférieure du piston (21) comporte le long de sa circonférence extérieure une gorge (49) recevant l'élément d'étanchéité (50).

12. Endoscope selon la revendication 8, dans lequel l'élément d'étanchéité (50) est formé par moulage sur pièce insérée, la partie d'extrémité inférieure du piston (21) étant utilisée comme pièce insérée.

FIG. 1

0 069 913

F I G.   2

F I G.   3

FIG. 4

FORCE
(OPTIONAL
SCALE )

NATURAL CONDITION

DEPRESSION

COMPRESSION AMOUNT
(OPTIONAL SCALE)

FIG. 5

# F I G. 6

# F I G. 7

# F I G. 8